# EUROPÄISCHE PATENTSCHRIFT

(11) **EP 0 817 594 B1**
(45) Veröffentlichungstag und Bekanntmachung des Hinweises auf die Patenterteilung: **05.06.2002**
(21) Anmeldenummer: 96903869.4
(22) Anmeldetag: 07.03.1996
(51) Int. Cl.: A61B 17/22

(54) **KATHETER ZUM ABLÖSEN VON ABNORMALEN ABLAGERUNGEN IN MENSCHLICHEN BLUTGEFÄSSEN**
CATHETER FOR DETACHING ABNORMAL DEPOSITS IN HUMAN BLOOD VESSELS
CATHETER PERMETTANT DE DETACHER DES DEPOTS ANORMAUX SITUES DANS DES VAISSEAUX SANGUINS CHEZ L'HOMME

(30) Priorität: 28.03.1995 CH 87395
(43) Veröffentlichungstag der Anmeldung: 14.01.1998
(73) Patentinhaber: Straub Medical AG, 7323 Wangs (CH)
(72) Erfinder: STRAUB, Immanuel, CH-7323 Wangs (CH); MOHR, Helmuth, CH-9470 Buchs (CH)
(74) Vertreter: Patentanwälte Schaad, Balass, Menzl & Partner AG
(86) Internationale Anmeldenummer: CH9600085
(87) Internationale Veröffentlichungsnummer: WO9629941

(56) Entgegenhaltungen:
- EP-A- 0 582 533
- WO-A-91/01114
- WO-A-92/07500
- US-A- 4 857 046
- US-A- 5 112 299
- US-A- 5 269 751
- US-A- 5 383 884

## Beschreibung

Die Erfindung bezieht sich auf einen Katheter der im Oberbegriff des Anspruchs 1 genannten Art, welcher auch unter der Bezeichnung Rotationskatheter bekannt ist.

Ein solcher Katheter dient insbesondere zum Behandeln von arteriellen Verschlusserkrankungen durch Abtragen und Zerkleinern von Stenosen und Thromben. Er wird in die Arterie oder Vene eingeführt und bis zu der verengten Stelle vorgeschoben, die zu behandeln ist. An seinem vorderen oder vorlaufenden Ende ist ein rotierend antreibbares Schneidwerkzeug angeordnet.

Bei einem aus der WO-A1-91/01114 bekannten Katheter gemäss dem Oberbegriff des Anspruchs 1 weist der innerhalb des Stators angeordnete Rotor an seiner der Vorschubrichtung des Katheters entgegengerichteten Stirnseite eine umfangsseitig verlaufende wellenförmige Schneide auf. Der Stator weist in seiner Mantelfläche einen Ausschnitt mit einer der Schneide des Rotors entgegengerichteten "schnabelartigen" Kante auf. Trifft der Katheter während seines Vorschubs auf der Seite des Ausschnitts im Stator auf Ablagerungen, dann dringen diese mindestens teilweise in den Ausschnitt hinein. Der Vorschub des Katheters muss dann unterbrochen und das Schneidwerkzeug muss mittels einer an der Gegenseite der Arterie oder Vene angreifenden, manuell betätigbaren Spannvorrichtung gegen die Ablagerungen vorgespannt werden. Dann muss der rotierend angetriebene Rotor mittels einer an seiner flexiblen Antriebswelle angreifenden Betätigungsvorrichtung von Hand gegen die Kante im Statorausschnitt zurückgezogen werden, während seine Schneide die in den Ausschnitt hineinragenden Ablagerungen abtrennt, wobei die Kante im Stator als Gegenhaltung wirkt. Der Schneidvorgang erfolgt demnach in Längsrichtung zum Katheter. Die abgetrennten Ablagerungen werden durch ein an den Katheterschlauch angelegtes Vakuum abgesaugt.

Dieser bekannte Katheter ist umständlich in der Handhabung und erlaubt keinen kontinuierlichen Vorschub. Zudem ist er mit dem Risiko behaftet, dass er mit der "schnabelartig" ausgebildeten Kante an den Ablagerungen rupft, wenn er zu weit vorgeschoben wird. Dabei ist eine Verletzung der Arterie oder Vene nicht auszuschliessen.

Ein anderer aus der EP-B1-0 267 539 bekannter Katheter weist als Schneidwerkzeug einen im wesentlichen ellipsenförmigen Schneidfräser auf, dessen Oberfläche mit abrasivem Material versehen ist und der mit einer Drehzahl bis zu 160'000/min angetrieben wird. Der Schneidfräser ist über eine biegsame Antriebswelle mit einem am anderen Ende des Katheters angeordneten Drehantrieb verbunden. Die Antriebswelle läuft in einer als Katheterschlauch dienenden schlauchförmigen Hülle. Durch die Antriebswelle hindurch erstreckt sich ein Führungsdraht, der vor dem Einführen des Katheters in die Arterie oder Vene eingeführt und vorgeschoben wird.

Bei diesem bekannten Rotationskatheter ist das Risiko nicht auszuschliessen, dass insbesondere in einer Krümmung die Gefässwand verletzt und unter Umständen sogar durchgefräst wird.

Ein weiterer bekannter Rotationskatheter weist ein Schneidwerkzeug mit zwei Schälmessern auf, welches mit einer Drehzahl von 750/min angetrieben wird. Bei diesem Katheter besteht das Risiko, dass die Schälmesser, insbesondere bei der relativ langsamen Umfangsgeschwindigkeit, rupfen, reissen oder sich mit der Gefässwand verklemmen können.

Der Erfindung liegt deshalb die Aufgabe zugrunde, einen Katheter der eingangs genannten Art zu schaffen, der einerseits ein sauberes Abtragen abnormaler Ablagerungen in menschlichen Blutgefässen gewährleistet und andererseits Verletzungen der Gefässwand mit grosser Wahrscheinlichkeit vermeidet.

Die gestellte Aufgabe wird erfindungsgemäss durch die im Anspruch 1 angegebenen Merkmale gelöst.

Der erfindungsgemässe Katheter gewährleistet, dass nur hervorstehende und zwischen die Schneidkanten gelangende Ablagerungen erfasst und abgetrennt werden können. Es ist dabei nahezu ausgeschlossen, dass durch das Schneidwerkzeug die Gefässwand verletzt werden kann. Auch das Risiko, dass das Schneidwerkzeug eines solchen Katheters an der Gefässwand reisst und rupft, ist durch die Scherwirkung in Verbindung mit der Gegenschneide praktisch ausgeschlossen.

Bei einer bevorzugten Ausführungsform nach Anspruch 2 greift der Rotor radial an den Ablagerungen an. Dadurch wird verhindert, dass beispielsweise im Bereich von Krümmungen geradeaus in die Gefässwand gebohrt werden kann.

Eine weitere Sicherheit gegen Verletzungen der Gefässwand bietet eine Ausführungsform nach Anspruch 3. Durch die Anordnung von Scherfenstern werden letztendlich nur solche Ablagerungen abgelöst, die in die Scherfenster hineinragen.

Eine Ausführungsform nach Anspruch 4 gewährleistet eine Symmetrie der Scherwirkung, da gleichzeitig an sich diametral gegenüberliegenden Stellen der Gefässwand angegriffen wird. Dies ergibt einen besseren Rundlauf des Rotors, als wenn dieser umfangsseitig nur an einer Stelle angreifen würde.

Anspruch 5 beschreibt eine bevorzugte Ausführungsform für die Anordnung der Schneiden. Es ist jedoch auch möglich, gerade verlaufende Schneiden schräg zur Achsrichtung oder in einer Ausführungsform nach Anspruch 6 anzuordnen.

Eine Ausführungsform nach Anspruch 7 gibt dem Rotor die Möglichkeit, bereits vor dem Einsetzen der Scherwirkung in das Scherfenster hineinragende bzw. hineinquellende Ablagerungen von der Gefässwand abzutrennen.

Ausführungsformen nach den Ansprüchen 8 bis 10 gewährleisten, dass sich der Rotor insbesondere an verengten bzw. verstopften Stellen einen Weg durch das Blutgefäss bahnt.

Anspruch 11 gibt eine bevorzugte Werkstoffauswahl an. Es ist jedoch auch möglich, andere Werkstoffe für diesen Zweck zu verwenden, beispielsweise geeignete Kunststoffe.

Eine Ausführungsform nach Anspruch 12 erleichtert die Einführung des Katheters in die Arterie oder Vene auf dem zuvor eingeführten Führungsdraht.

Anspruch 13 beschreibt eine bevorzugte Ausführungsform zur Befestigung des Stators. Es ist jedoch auch möglich, den Stator an der Spitze bzw. am vorlaufenden Ende der schlauchförmigen Hülle beweglich zu befestigen.

Bevorzugte Ausführungsformen nach den Ansprüchen 14 und 15 gewährleisten einen Abtrag durch die Schneidwerkzeuge über den vollen Umfang der Gefässwand, wobei der Stator derart bewegt wird, dass die in ihm angeordneten Scherfenster entweder eine langsam umlaufende oder reversierbare Schwenkbewegungen um die Längsachse des Stators ausführen. Bei einer solchen Bewegung führt der Stator während des Vorschubs entweder eine fortlaufend schraubenförmige oder jeweils abwechselnd links- und rechtsgerichtete schraubenförmige Bewegungen aus. Im einfachsten Fall kann eine solche Bewegung manuell durch den behandelnden Arzt erfolgen, wenn der Stator dabei nach Anspruch 13 mit der schlauchförmigen Hülle dreh- und zugfest verbunden ist.

Bei einer bevorzugten Ausführungsform nach Anspruch 16 ist eine gleichmässig umlaufende oder reversierbare Bewegung des Stators gewährleistet. Der dazu erforderliche Schwenkantrieb kann bei einer Ausführung nach Anspruch 13 mit dem herausragenden hinteren Ende der schlauchförmigen Hülle verbunden sein oder als Miniaturausführung unmittelbar am Stator angreifen. Es ist auch möglich, zwischen dem Rotor und dem Stator ein Miniatur-Untersetzungsgetriebe anzuordnen, um den Stator durch die Drehbewegung des Rotors anzutreiben, vorzugsweise gegenüber dem Rotor in entgegengesetzter Drehrichtung.

Anspruch 17 beschreibt eine bevorzugte Ausführungsform für eine besonders einfache dreh- und zugfeste Befestigungsart des Stators an der schlauchförmigen Hülle.

Anspruch 18 beschreibt eine bevorzugte Ausführungsform für den Antrieb des Rotors. Es ist aber auch möglich, den Rotor mittels eines Miniaturgetriebes unmittelbar anzutreiben.

Eine bevorzugte Ausführungsform nach Anspruch 19 ermöglicht einen sofortigen Wegtransport der abgelösten und zerkleinerten Ablagerungen, um zu vermeiden, dass diese im Blutkreislauf verbleiben.

Durch eine Ausführungsform nach Anspruch 20 wird der Wirkungsgrad der Förderschraube erhöht.

Anhand der Zeichnungen wird ein Ausführungsbeispiel der Erfindung näher erläutert. Es zeigen:
- Fig. 1: Einen Rotationskatheter in Gesamtansicht, mit Antrieb, Führungsdraht und Auffanggefäss für die abgelösten Ablagerungsteile,
- Fig. 2: den Kopfteil des Rotationskatheters nach Fig. 1, jedoch in einem grösseren Massstab, im Aufriss,
- Fig. 3: den Kopfteil wie Fig. 2, jedoch im Grundriss,
- Fig. 4: eine Stirnansicht auf den Rotor und den Führungsdraht des Rotationskatheters nach Fig. 3,
- Fig. 5: den Kopfteil im Querschnitt entlang der Schnittlinie V - V nach Fig. 3,
- Fig. 6: den Rotationskatheter nach Fig. 3, wobei jedoch der Rotor relativ zum Stator um 90° verdreht dargestellt ist,
- Fig. 7: einen Längsschnitt durch den Kopfteil des Rotationskatheters gemäss Fig. 2,
- Fig. 8: Führungsdraht und Wendel in einem Querschnitt durch den Wendel,
- Fig. 9: den Kopfteil des Katheters nach Fig. 1 in perspektivischer Ansicht, mit Blickrichtung auf die vordere Stirnseite,
- Fig. 10: den Kopfteil des Katheters nach Fig. 1 in perspektivischer Ansicht, von der Antriebsseite gesehen,

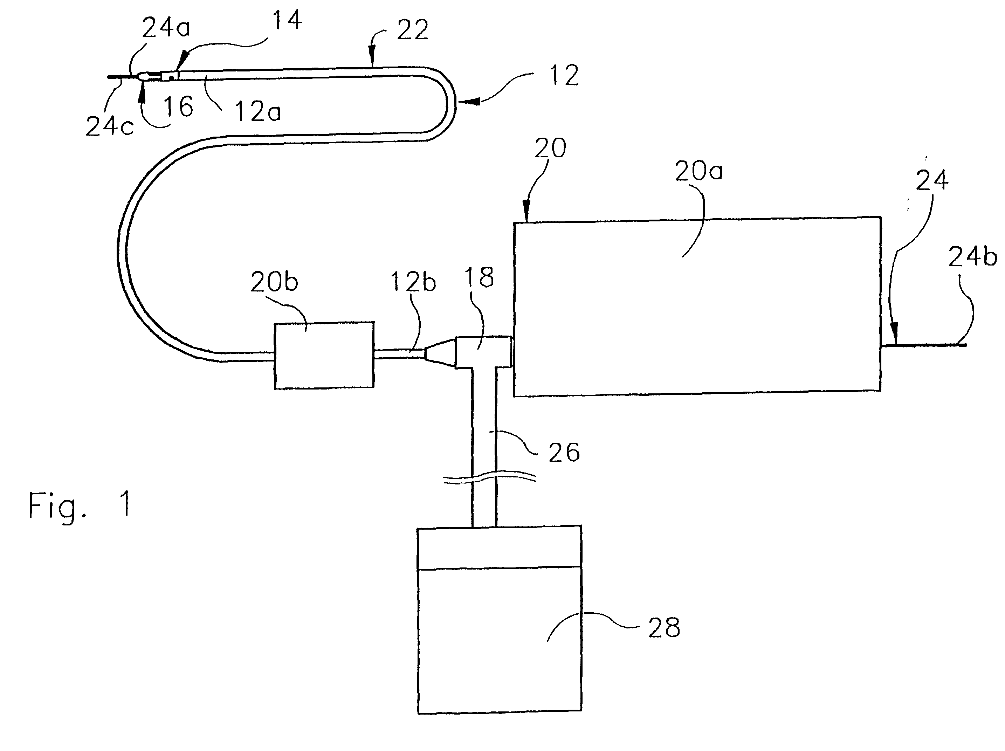

Der in Fig. 1 dargestellte Katheter **12,** weist an seinem vorderen Ende **12a** ein aus Stator **14** und Rotor **16** bestehendes Schneidwerkzeug auf. An seinem hinteren Ende **12b** ist der Katheter **12** über eine Abfuhrkammer **18** mit einem Drehantrieb **20a** einer Antriebseinheit **20** verbunden. In einer als Katheterschlauch dienenden schlauchförmigen Hülle **22** ist eine flexible Antriebswelle gelagert, die den Rotor **16** mit dem Drehantrieb **20a** verbindet. Durch die gesamte Länge des Katheters **12** hindurch erstreckt sich ein Führungsdraht **24**, dessen vorderes Ende **24a** aus dem Rotor **16** und dessen hinteres Ende **24b** aus der Antriebseinheit **20** herausragt. An die Abfuhrkammer **18** ist in radialer Richtung über einen Schlauch oder ein Rohr **26** ein Auffanggefäss **28** angeschlossen.

Die schlauchförmige Hülle **22** ist mit einem Schwenkantrieb **20b** drehfest verbunden. Dieser kann entweder für eine umlaufende oder für eine reversierbare Schwenkbewegung beschaffen sein. Seine Drehzahl liegt wesentlich unterhalb derjenigen des Drehantriebes **20a**.

Der Schwenkantrieb **20b** kann auch weggelassen werden, wenn lediglich die schlauchförmige Hülle **22** drehbar gelagert ist. Bei einer solchen Ausführung kann die schlauchförmige. Hülle manuell in eine umlaufende oder hin- und hergehende Schwenkbewegung versetzt werden, wenn der Katheter **12** bei seinem Vorschub die zu behandelnden Stelle erreicht hat.

Es ist auch möglich, den Stator **14** von der schlauchförmigen Hülle **22** zu entkoppeln und nur den Stator **14** schwenkbar zu lagern und unmittelbar mit einem nicht dargestellten Miniatur-Schwenkantrieb auszurüsten.

Bei der Anwendung des Katheters **12** wird der Führungsdraht **24** unter Röntgenkontrolle mit seinem vorderen Ende **24a** voraus in die zu behandelnde Arterie oder Vene bis zur verengten Stelle eingeführt und dann durch diese hindurch manövriert. Anschliessend wird der Katheter **12** über den Führungsdraht **24** nachgeführt. Sobald der Rotor **16** die zu behandelnde Stelle erreicht hat, wird mindestens der Drehantrieb **20a** eingeschaltet, um mittels des Schneidwerkzeuges die unerwünschten Ablagerungen abzulösen. Die Drehzahl des Rotors **16** liegt vorzugsweise im Bereich zwischen 30'000 und 40'000/min. Während des Betriebes wird der Katheter **12** langsam vorgeschoben und dabei entweder mittels des Schwenkantriebes **20b** oder von Hand in eine langsame Schwenkbewegung versetzt. Die abgetrennten und zerkleinerten Ablagerungen werden durch die schlauchförmige Hülle **22** bis zur Abfuhrkammer **18** weggefördert und gelangen von dort in das Auffanggefäss **28.**

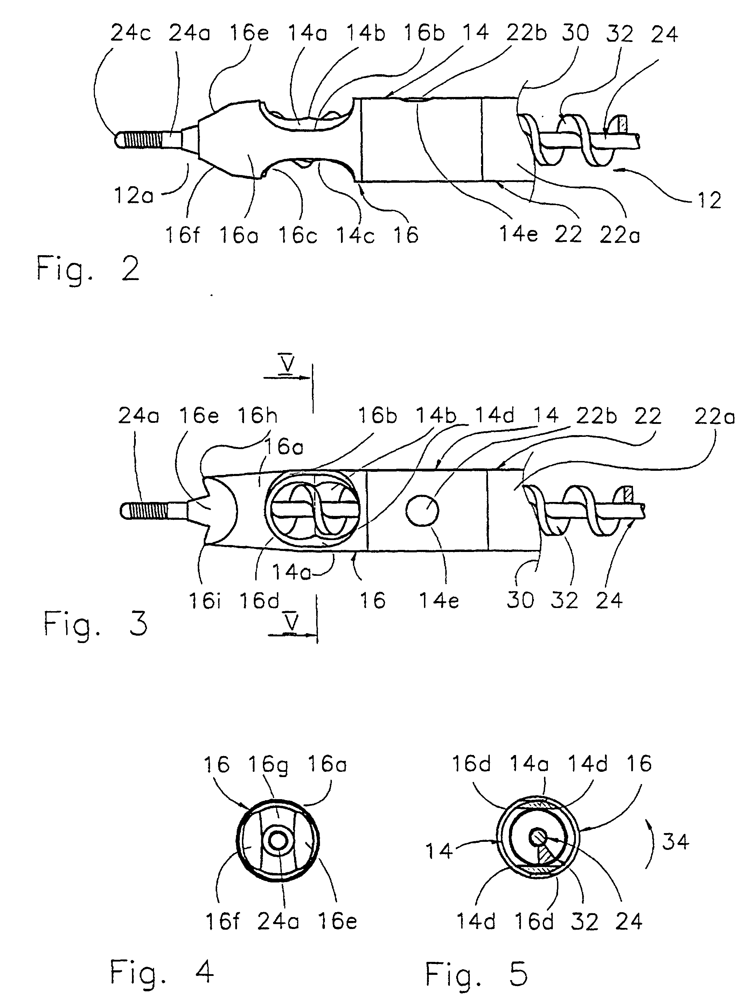

Die Fig. 2 zeigt das vordere Ende **12a** des Katheters **12** mit seinem Stator **14**, seinem als Aussenläufer ausgebildetem Rotor **16**, seiner schlauchförmigen Hülle **22** sowie dem vorderen Ende **24a** des Führungsdrahtes **24**. Die durch den Schnitt **30** unterbrochen dargestellte schlauchförmige Hülle **22** lässt die flexible Antriebswelle **32** erkennen, die innerhalb des Rotors **16** mit diesem drehfest verbunden ist. Innerhalb der Antriebswelle **32** erstreckt sich der Führungsdraht **24.** Die Antriebswelle **32** ist zusätzlich als Förderschnecke bzw. Förderschraube ausgebildet, um die durch das Schneidwerkzeug **14**, **16** abgetragenen Ablagerungen durch die schlauchförmige Hülle **22** zur Abfuhrkammer **18** zu fördern.

Ein Abschnitt **14a** des Stators **14** erstreckt sich in den Rotor **16** hinein. Es ist ersichtlich, dass der Statorabschnitt **14a** und der Rotor **16** hülsenförmig ineinandergreifen. Der Statorabschnitt **14a** weist zwei umfangsseitig um 180° zueinander versetzte Scherfenster **14b, 14c** auf. Der Rotor **16** weist ebenfalls zwei umfangsseitig um 180° zueinander versetzte Fenster **16b, 16c** auf.

Aus der Fig. 3 ist ersichtlich, dass das Scherfenster **14b** des Statorabschnitts **14a** in Umfangsrichtung schmäler als dasjenige **16b** des Rotors **16** ist. Die eine Kante des Rotorfensters **16b** ist als Schneide **16d** ausgebildet. Die entgegengerichtete Kante des Statorfensters **14b** ist als Schneide **14d** ausgebildet. Diese Schneide **14d** verläuft mindestens annähernd wellenförmig.

Die Schneide **16d** und die Schneide **14d** wirken scherenartig zusammen. Derartige Schneiden sind jeweils in beiden, auch als Scherfenster zu bezeichnenden Fenstern **14b, 14c; 16b, 16c**, also umfangsseitig um 180° nacheinander angeordnet. Das vordere Ende **16a** des Rotors **16** ist mindestens annähernd konisch verjüngt. Dadurch wird die verengte und zu behandelnde Stelle der Arterie oder Vene beim Einführen des Katheters **12** aufgeweitet.

Die Fig. **4** zeigt die Stirnansicht des Rotors **16** und des vorderen Endes **24a** des Führungsdrahtes **24**. Ferner ersichtlich sind zwei einander entgegengesetzte Schrägflächen **16e**, **16f** des Rotors **16**, zwischen denen sich eine Stirnfläche **16g** befindet. Die Stirnfläche **16g** weist umfangsseitig hornähnliche, sich nach vorn erstreckende Vorsprünge **16h, 16i** auf (Fig. 3). Die Stirnseite des Rotors **16** dient insbesondere zum Zerschlagen bzw. Zerkleinern von den Durchlass versperrenden Thromben, um dem Katheter **12** den Weg längs des Blutgefässes zu bahnen.

Die Fig. 5 zeigt einen Querschnitt gemäss V-V nach Fig. 3. Der Rotor **16** wird in Pfeilrichtung **34** angetrieben. Dabei greifen die Schneiden **16d** des Rotors **16** umfangsseitig an den Ablagerungen, beispielsweise an den Stenosen, an und zerkleinern diese. Die Schneiden **14d** des Statorabschnitts **14a** erzielen zusammen mit den Rotorschneiden **16d** eine Scherwirkung, wobei die abgescherten Teile der Ablagerungen in den Bereich der Antriebswelle **32** bzw. Förderschraube gelangen und von dieser bis zur Abfuhrkammer **18** (Fig. 1) wegtransportiert werden. Bei dieser Darstellung ist zu beachten, dass der Aussendurchmesser des Rotors **16** weniger als 3 mm beträgt.

Der Rotor **16** und der Stator **14** bestehen vorzugsweise aus Metall. Der Führungsdraht **24** ist ein Stahldraht mit Federspitze **24c.** Die auch als Förderschnecke bzw. Förderschraube dienende Antriebswelle **32** besteht beispielsweise aus einem beschichteten Stahldraht. Die schlauchförmige Hülle **22** besteht vorzugsweise aus Kunststoff.

Zur drehfesten Verbindung des Stators **14** mit der schlauchförmigen Hülle **22** wird deren vorderes Ende **22a** (Fig. 2 und 3) beispielsweise in den Stator **14** hineingepresst. Zur Fixierung sind in der Mantelfläche des Stators **14** Löcher **14e** angeordnet, in die das eingepresste Schlauchmaterial **22b** geringfügig hineinquillt.

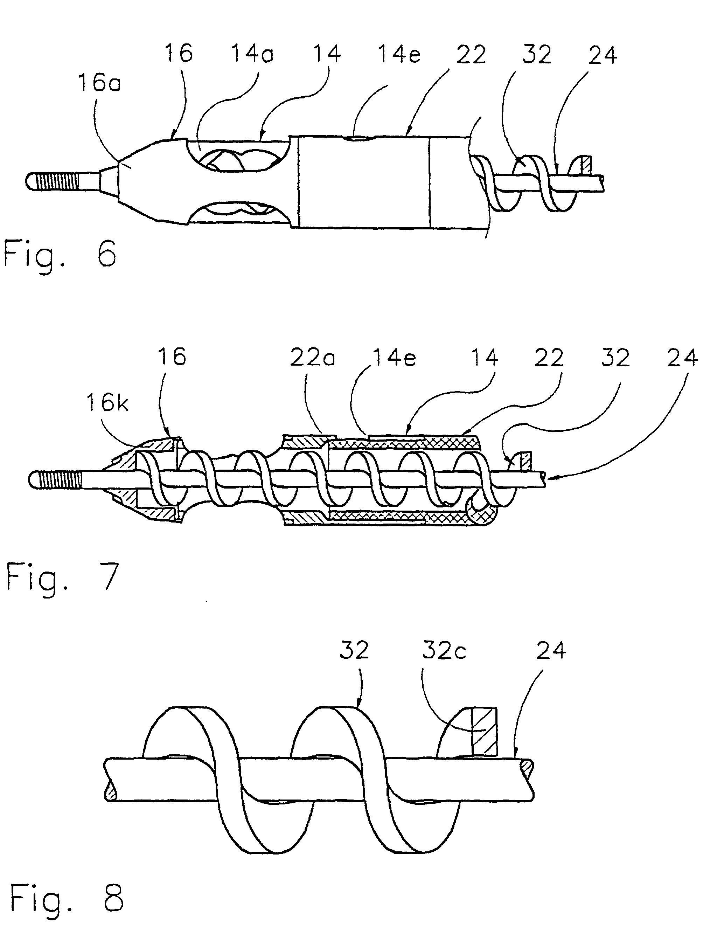

Bei der Ansicht nach der Fig. 6 entspricht die Stellung des Stators **14** derjenigen in Fig. 3 und die Stellung des Rotors **16** derjenigen in Fig. 2. Dabei wird der geringfügige Durchmesser-Unterschied zwischen dem Statorabschnitt **14a** und dem Rotor **16** deutlich sichtbar.

Aus dem Längsschnitt nach der Fig. 7 ist insbesondere ersichtlich, dass sich die Antriebswelle **32** mit ihrem vorderen Ende **32a** bis in den Kopfteil **16k** des Rotors **16** hineinerstreckt und dort mit diesem drehfest verbunden, beispielsweise in diesen eingepresst ist. Ebenfalls ersichtlich ist, wie die schlauchförmige Hülle **22** im Stator **14** durch die Löcher **14e** zug- und drehfest gesichert ist.

Die Fig. **8** zeigt insbesondere den rechteckigen Drahtquerschnitt **32c** der wendelförmigen Antriebswelle **32,** die gleichzeitig auch als Förderschnecke oder Förderschraube dient. Durch die Anordnung des Führungsdrahtes **24** koaxial innerhalb der Antriebswelle **32** ergibt sich ein besonders guter Wirkungsgrad als Förderschnecke oder Förderschraube. Die Förderung der abgetragenen Teile der Ablagerungen erfolgt nahezu linear innerhalb der schlauchförmigen Hülle **22**.

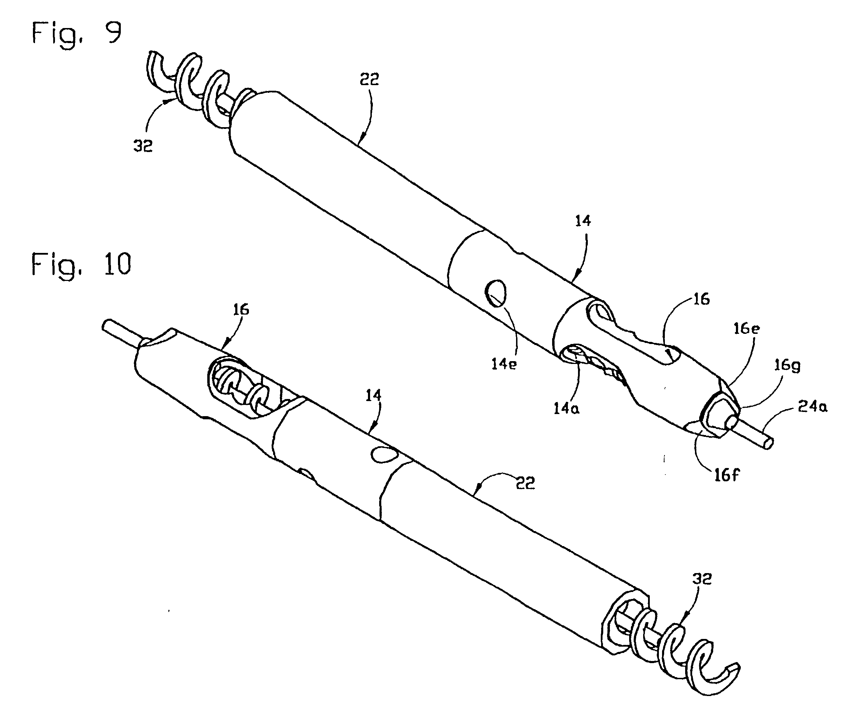

Die Figuren 9 und 10 zeigen alle bereits beschriebenen Teile, jedoch in perspektivischer Darstellung.

## Patentansprüche

1. Katheter zum Ablösen von abnormalen Ablagerungen in menschlichen Blutgefässen, mit einem an seinem vorderen Ende (**12a**) angeordneten Schneidwerkzeug, welches einen Stator (**14**) und einen mittels eines Drehantriebs (**20a**) einer Antriebseinheit (**20**) in Rotation versetzbaren Rotor (**16**) aufweist, der in der Mantelfläche mit einer Schneide (**16d**) ausgerüstet ist, sowie mit einer schlauchförmigen Hülle **(22)** zur Abfuhr der abgelösten Ablagerungen, **dadurch gekennzeichnet, dass** der Rotor **(16)** einen Abschnitt **(14a)** des Stators **(14)** als Aussenläufer umgibt und dass in der Mantelfläche des Statorabschnitts (**14a**) mindestens eine Gegenschneide (**14d**) angeordnet ist, die mit der Schneide (**16d**) des Rotors **(16)** scherenartig zusammenwirkt.

2. Katheter nach Anspruch 1, **dadurch gekennzeichnet, dass** der Rotor (**16**) und der Abschnitt (**14a**) des Stators (**14**) mindestens im Bereich der Schneiden (**14d, 16d**) zylinderförmig sind.

3. Katheter nach Anspruch 1 oder 2, **dadurch gekennzeichnet, dass** in den Mantelflächen des Statorabschnitts **(14a)** und des Rotors **(16)** Scherfenster **(14b, 14c; 16b, 16c)** angeordnet sind, deren Kanten als die Schneiden **(14d, 16d)** ausgebildet sind.

4. Katheter nach Anspruch 3, **dadurch gekennzeichnet, dass** der Statorabschnitt **(14a)** und der Rotor **(16)** je zwei in Umfangsrichtung um 180° zueinander versetzt angeordnete Scherfenster (**14b, 14c; 16b, 16c**) aufweisen.

5. Katheter nach einem der vorstehenden Ansprüche, **dadurch gekennzeichnet, dass** sich die Schneiden (**14d, 16d**) mindestens annähernd in Achsrichtung erstrecken.

6. Katheter nach einem der vorstehenden Ansprüche, **dadurch gekennzeichnet, dass** mindestens eine der Schneiden (**14d**), bezogen auf eine zylindrische Fläche, in axialer Richtung wellenförmig ist.

7. Katheter nach einem der vorstehenden Ansprüche, **dadurch gekennzeichnet, dass** die Schneiden (**16d**) des Rotors (**16**) Messerschneiden sind.

8. Katheter nach einem der vorstehenden Ansprüche, **dadurch gekennzeichnet, dass** der Rotor (**16**) in Richtung seines vorderen Endes (**16a**) mindestens teilweise verjüngt ist.

9. Katheter nach Anspruch 8, **dadurch gekennzeichnet, dass** der Rotor (**16**) an seiner Stirnseite Mittel (**16e, 16f, 16g, 16h, 16i**) zum Zerschlagen bzw. Zerkleinern von losen und festen Ablagerungen, beispielsweise Thromben, aufweist.

10. Katheter nach Anspruch 9, **dadurch gekennzeichnet, dass** die Mittel eine Stirnfläche (**16g**) und zwei angrenzende, sich gegenüberliegende Schrägflächen (**16e, 16f**) aufweisen und dass die Stirnfläche (**16g**) umfangsseitig hornähnliche, sich nach vorn erstreckende Vorsprünge (**16h, 16i**) aufweist.

11. Katheter nach einem der vorstehenden Ansprüche, **dadurch gekennzeichnet, dass** der Stator (**14**) und/oder der Rotor (**16**) aus Metall bestehen.

12. Katheter nach einem der vorstehenden Ansprüche, **dadurch gekennzeichnet, dass** sich innerhalb der flexiblen Antriebswelle **(32)** vorzugsweise ein vom Katheter unabhängiger Führungsdraht **(24)** durch den Katheter **(12)** hindurcherstreckt.

13. Katheter nach einem der vorstehenden Ansprüche, **dadurch gekennzeichnet, dass** der Stator (**14**) mit der als Katheterschlauch dienenden schlauchförmigen Hülle (**22**) dreh- und zugfest verbunden ist.

14. Katheter nach einem der vorstehenden Ansprüche, **dadurch gekennzeichnet, dass** der Stator (**14**) um seine Längsachse umlaufend oder hin- und herschwenkbar gelagert ist.

15. Katheter nach Anspruch 14, **dadurch gekennzeichnet, dass** der Schwenkwinkel des Stators (**14**) so gross bemessen ist, dass durch das Schneidwerkzeug (**14d, 16d**) mindestens ein Abtragswinkel von 360° erfasst wird.

16. Katheter nach Anspruch 14 oder 15, **dadurch gekennzeichnet, dass** der Stator (**14**) oder die schlauchförmige Hülle (**22**) mit einem Schwenkantrieb (**20b**) verbunden ist, dessen Ausgangsdrehzahl wesentlich unter derjenigen des Drehantriebes (**20a**) liegt.

17. Katheter nach einem der Ansprüche 13 bis 16, **dadurch gekennzeichnet, dass** der hülsenförmige Stator (**14**) an seinem der schlauchförmigen Hülle (**22**) benachbarten Ende in seiner Mantelfläche mindestens ein Loch (**14e**) aufweist, um die aus einem Kunststoff bestehende, in den Stator (**14**) eingepresste Hülle (**22**) dreh- und zugfest zu verankern.

18. Katheter nach einem der vorstehenden Ansprüche, **dadurch gekennzeichnet, dass** der Rotor (**16**) mit dem vorderen Ende (**32a**) einer in der schlauchförmigen Hülle (**22**) gelagerten, flexiblen Antriebswelle (**32**) verbunden ist, deren hinteres Ende mit dem Drehantrieb (**20a**) verbindbar ist.

19. Katheter nach Anspruch 18, **dadurch gekennzeichnet, dass** die flexible Antriebswelle (**32**) als Förderschnecke oder Förderschraube ausgebildet und derart gewendelt ist, dass sie in angetriebenem Zustand die zerkleinerten Ablagerungen in Richtung der Antriebseinheit (**20**) fördert.

20. Katheter nach Anspruch 19, **dadurch gekennzeichnet, dass** sich ein Führungsdraht **(24)** koaxial durch die als Förderschnecke oder Förderschraube ausgebildete flexible Antriebswelle **(32)** hindürcherstreckt.

## Claims

1. Catheter for detaching abnormal deposits from blood vessels in humans, with a cutting tool which is arranged at its front end (12a) and which has a rotor (16) which can be set in rotation by means of a rotary drive mechanism (20a) of a drive unit (20), and with a tubular sheath (22) for discharging the deposits which have been detached, **characterized in that** the cutting tool has a stator (14) with a stator portion (14a), and **in that** cutting edges (14d, 16d) are arranged on the stator portion (14a) and on the rotor (16) to interact in a shearing action.

2. Catheter according to Claim 1, **characterized in that** the stator portion (14a) and the rotor (16) are at least approximately cylindrical, at least in the region of the cutting edges (14d, 16d), **in that** the rotor (16) surrounds the stator portion (14a) as external rotor, and **in that** the cutting edges (14d, 16d) are arranged in the circumferential surfaces of the rotor (16) and of the stator portion (14a).

3. Catheter according to Claim 2, **characterized in that** shearing slots (14b, 14c; 16b, 16c) are arranged in the circumferential surfaces of the stator portion (14a) and of the rotor (16), their margins being designed as the cutting edges (14d, 16d).

4. Catheter according to Claim 3, **characterized in that** the stator portion (14a) and the rotor (16) have in each case two shearing slots (14b, 14c; 16b, 16c) which are offset by 180° to each other in the circumferential direction.

5. Catheter according to one of the preceding claims, **characterized in that** the cutting edges (14d, 16d) extend at least approximately in the axial direction.

6. Catheter according to one of the preceding claims, **characterized in that** at least one of the cutting edges (14d) runs in an undulating configuration in the axial direction, relative to a cylindrical surface.

7. Catheter according to one of the preceding Claims, **characterized in that** the cutting edges (16d) of the rotor (16) are knife edges.

8. Catheter according to one of the preceding claims, **characterized in that** the rotor (16) is at least partially tapered in the direction of its front end (16a).

9. Catheter according to Claim 8, **characterized in that** the rotor (16) has, at its front, means (16e, 16f, 16g, 16h, 16i) for breaking up loose and solid deposits, for example blood clots.

10. Catheter according to Claim 9, **characterized in that** the means include one front face (16g) and two adjoining bevelled surfaces (16e, 16f) lying opposite each other, and **in that** the front face (16g) has, on the circumference, horn-like, forwardly extending projections (16h, 16i).

11. Catheter according to one of the preceding claims, **characterized in that** the stator (14) and/or the rotor (16) are made of metal.

12. Catheter according to one of the preceding claims, **characterized in that**, inside the flexible drive shaft (32), a guide wire (24) which is preferably independent of the catheter extends through the said catheter (12).

13. Catheter according to one of the preceding claims, **characterized in that** the stator (14) is connected, in a manner fixed in terms of rotation and tensioning, to the tubular sheath (22) serving as catheter tube.

14. Catheter according to one of the preceding claims, **characterized in that** the stator (14) is mounted such that it revolves or can swivel to and fro about its longitudinal axis.

15. Catheter according to Claim 14, **characterized in that** the swivel angle of the stator (14) is so great that at least an angle of removal of 360° is covered by the cutting tool (14d, 16d).

16. Catheter according to Claim 14 or 15, **characterized in that** the stator (14) or the tubular sheath (22) is connected to a swivel drive mechanism (20b) whose output speed is substantially below that of the rotary drive mechanism (20a).

17. Catheter according to one of Claims 13 to 16, **characterized in that** the sleeve-like stator (14) has in its circumferential surface, at its end adjacent to the tubular sheath (22), at least one hole (14e) for anchoring the sheath (22), which is made of plastic and is press-fitted into the stator (14), in a manner fixed in terms of rotation and tensioning.

18. Catheter according to one of the preceding claims, **characterized in that** the rotor (16) is connected to the front end (32a) of a flexible drive shaft (32) mounted in the tubular sheath (22), the rear end of which drive shaft (32) can be connected to the rotary drive mechanism (20a).

19. Catheter according to Claim 18, **characterized in that** the flexible drive shaft (32) is designed as a conveyor worm or conveyor screw and is wound helically in such a way that, in the driven state, it conveys the broken-up deposits in the direction of the rotary drive mechanism (20a).

20. Catheter according to Claim 19, **characterized in that** a guide wire (24) extends coaxially through the flexible drive shaft (32) designed as conveyor worm or conveyor screw.

## Revendications

1. Cathéter destiné à détacher des dépôts anormaux dans des vaisseaux sanguins humains, comportant un outil de coupe agencé à son extrémité antérieure (12a), lequel présente un stator (14) et un rotor (16) qui peut être mis en rotation au moyen d'un entraînement rotatif (20a) d'une unité d'entraînement (20), ledit rotor étant équipé d'un tranchant (16d) dans sa surface enveloppe, ainsi qu'un manchon (22) tubulaire pour évacuer les dépôts détachés, **caractérisé en ce que** le rotor (16) entoure, en tant qu'induit extérieur, un tronçon (14a) du stator (14) et **en ce que** dans la surface enveloppe du tronçon de stator (14a) est agencé au moins un contre-tranchant (14d) qui coopère à la manière de ciseaux avec le tranchant (16d) du rotor (16).

2. Cathéter selon la revendication 1, **caractérisé en ce que** le rotor (16) et le tronçon (14a) du stator (14) sont de forme cylindrique au moins dans la région des tranchants (14d, 16d).

3. Cathéter selon l'une ou l'autre des revendications 1 et 2, **caractérisé en ce que** dans les surfaces enveloppes du tronçon de stator (14a) et du rotor (16) sont agencées des fenêtres de cisaillement (14b, 14c ; 16b, 16c) dont les arêtes sont réalisées à titre des tranchants.

4. Cathéter selon la revendication 3, **caractérisé en ce que** le tronçon de stator (14a) et le rotor (16) présentent chacun deux fenêtres de cisaillement (14b, 14c ; 16b, 16c) agencées en décalage mutuel de 180° en direction circonférentielle.

5. Cathéter selon l'une quelconque des revendications précédentes, **caractérisé en ce que** les tranchants (14d, 16d) s'étendent au moins approximativement en direction axiale.

6. Cathéter selon l'une quelconque des revendications précédentes, **caractérisé en ce qu'**au moins un des tranchants (14d) a une forme ondulée en direction axiale, par rapport à une surface cylindrique.

7. Cathéter selon l'une quelconque des revendications précédentes, **caractérisé en ce que** les tranchants (16d) du rotor sont des tranchants de couteaux.

8. Cathéter selon l'une quelconque des revendications précédentes, **caractérisé en ce que** le rotor (16) est rétréci au moins partiellement en direction de son extrémité antérieure (16a).

9. Cathéter selon la revendication 8, **caractérisé en ce que** le rotor (16) présente sur sa face frontale des moyens (16e, 16f, 16g, 16h, 16i) pour briser ou concasser des dépôts flottants ou stationnaires, par exemple des thrombus.

10. Cathéter selon la revendication 9, **caractérisé en ce que** les moyens présentent une face frontale (16g) et deux surfaces obliques (16e, 16f) adjacentes opposées l'une à l'autre et **en ce que** la face frontale (16g) présente du côté périphérique des saillies (16h, 16i) en forme de cornes qui s'étendent vers l'avant.

11. Cathéter selon l'une quelconque des revendications précédentes, **caractérisé en ce que** le stator (14) et/ou le rotor (16) sont en métal.

12. Cathéter selon l'une quelconque des revendications précédentes, **caractérisé en ce qu'**à l'intérieur de l'arbre moteur (32) flexible, un fil de guidage (24) indépendant du cathéter s'étend à travers le cathéter (12).

13. Cathéter selon l'une quelconque des revendications précédentes, **caractérisé en ce que** le stator (14) est relié de manière solidaire en rotation et en traction avec le manchon (22) tubulaire servant de tube de cathéter.

14. Cathéter selon l'une quelconque des revendications précédentes, **caractérisé en ce que** le stator (14) est monté en rotation autour de son axe longitudinal ou de manière à pivoter en va-et-vient.

15. Cathéter selon la revendication 14, **caractérisé en ce que** l'angle de pivotement du stator (14) a des dimensions telles qu'au moins un angle de creusement de 360° est balayé par l'outil coupant (14d, 16d).

16. Cathéter selon l'une ou l'autre des revendications 14 et 15, **caractérisé en ce que** le stator (14) ou le manchon (22) tubulaire est relié à un entraînement pivotant (20b) dont la vitesse de sortie est sensiblement inférieure à celle de l'entraînement rotatif (20a).

17. Cathéter selon l'une quelconque des revendications 13 à 16, **caractérisé en ce que** le stator (14) en forme de manchon présente dans sa surface enveloppe, à son extrémité voisine du manchon tubulaire (22), au moins un trou (14e) pour ancrer de manière solidaire à la rotation et à la traction le manchon (22) en matière plastique pressé dans le stator (14).

18. Cathéter selon l'une quelconque des revendications précédentes, **caractérisé en ce que** le rotor (16) est relié avec l'extrémité antérieure (32a) d'un arbre moteur (32) flexible monté dans le manchon tubulaire (22), dont l'extrémité postérieure peut être reliée avec l'entraînement rotatif (20a).

19. Cathéter selon la revendication 18, **caractérisé en ce que** l'arbre d'entraînement (32) flexible est réalisé sous forme d'hélice transporteuse ou de vis sans fin et **en ce qu'**il est spiralé de telle sorte qu'à l'état entraîné, il transporte les dépôts concassés en direction de l'unité d'entraînement (20).

20. Cathéter selon la revendication 19, **caractérisé en ce qu'**un fil de guidage (24) s'étend coaxialement à travers l'arbre moteur (32) flexible réalisé sous forme d'hélice transporteuse ou de vis sans fin.
